# EUROPEAN PATENT APPLICATION

(11) **EP 4 810 022 A1**
(43) Date of publication of application: **23.09.2026**
(21) Application number: 25739125.0
(22) Date of filing: 10.01.2025
(51) Int. Cl.: A61K 9/16, A61K 9/00, A61K 38/22

(54) **MICROPARTICLES COMPRISING TIRZEPATIDE OR PHARMACEUTICALLY ACCEPTABLE SALT THEREOF, AND MANUFACTURING METHOD THEREFOR**

(30) Priority: 10.01.2024 KR 20240004289
(71) Applicant: Inventage Lab Inc., Gyeonggi-do 13403 (KR)
(72) Inventor: KIM, Ju Hee, Seongnam-si, Gyeonggi-do 13494 (KR); LEE, Sang No, Seongnam-si, Gyeonggi-do 13494 (KR)
(74) Representative: BCKIP Part mbB
(86) International application number: PCT/KR2025/000604
(87) International publication number: WO 2025/150965

(57) **Abstract**

The present invention relates to microparticles comprising tirzepatide or a pharmaceutically acceptable salt thereof and a manufacturing method therefor, wherein the content of tirzepatide or a pharmaceutically acceptable salt thereof is increased in the microparticles, thereby increasing the loading amount of a drug in the microparticles and exhibiting an effect of continuously releasing tirzepatide or a pharmaceutically acceptable salt thereof from the microparticles without initial over-release and lag time, thus preventing an excessive decrease in bioavailability. In addition, when the microparticles are used as a subcutaneous injection, the amount of microparticles included for one injection can be reduced, thereby reducing pain at the site of administration and causing no inflammatory response.

## Description

### Technical Field

The present invention relates to microparticles containing tirzepatide or a pharmaceutically acceptable salt thereof and a method for producing the same.

### Background Art

Recently, due to economic development and changes in dietary habits, the incidence of metabolic syndrome-related diseases, including various diseases such as obesity, hyperlipidemia, hypertension, arteriosclerosis, hyperinsulinemia, diabetes, and liver disease, has rapidly increased. While these diseases may occur individually, they are usually closely related to each other and are often accompanied by various symptoms.

Overweight and obesity increase blood pressure and cholesterol levels, which causes the onset or worsening of various diseases such as heart disease, diabetes, and arthritis. In addition, overweight and obesity are major factors that increase the incidence of arteriosclerosis, hypertension, hyperlipidemia, and heart disease not only in adults but also in children and adolescents.

Since obesity is a complex disease associated with the mechanisms of appetite control and energy metabolism, methods of treating abnormal mechanisms of action related to appetite control and energy metabolism are required to be performed simultaneously, and thus efforts have been made to develop a drug capable of treating the abnormal mechanisms of action. As a result of the efforts, anti-obesity drugs such as rimonabant (Sanofi-Aventis), sibutramine (Abbott), contrave (Takeda), orlistat (Roche) have been developed, but these drugs have the disadvantages of showing fatal side effects or having insufficient effects on the treatment of obesity. For example, it has been reported that rimonabant shows central nervous system side effects, sibutramine and contrave show cardiovascular side effects, and orlistat has the effect of showing a body weight loss of only about 4 kg when taken for one year.

Meanwhile, metabolic syndromes, including obesity, increase the likelihood of causing diseases even in the liver. Examples thereof include metabolic liver disease, fatty liver, non-alcoholic fatty liver disease, steatohepatitis, liver fibrosis, etc. These liver diseases are on the rise as the obese and diabetic populations increase, and the annual incidence rate thereof in Korea is about 16%. Liver disease has no noticeable symptoms at the early stages and is discovered only after it has progressed significantly, and thus is a leading cause of death not only in Korea but also globally, and the development of drugs for liver disease is highly required.

For the treatment of obesity, etc., glucagon-like peptide-1 (GLP-1) is a hormone secreted from the small intestine in response to food intake, and helps lower blood glucose levels by promoting insulin secretion from the pancreas in a blood glucose level-dependent manner and inhibiting the secretion of glucagon. In addition, it acts as a satiety factor, slowing down the digestion of food in the stomach and delaying the stomach transit time of digested food, thereby reducing food intake.

GIP, which is one of the gastrointestinal hormones secreted in response to food intake along with GLP-1, is a 42-amino-acid hormone secreted from K cells in the small intestine, and performs the function of promoting insulin secretion from the pancreas in a blood glucose level-dependent manner and helping to lower blood glucose levels. GIP has been reported to have the effect of increasing the activity of GLP-1, anti-inflammatory effects, the effect of improving lipid metabolism, and the like.

Accordingly, studies have been actively conducted to develop GLP-1 as a therapeutic agent for diabetes and obesity by utilizing the effects thereof on blood glucose level control and weight loss. However, GLP-1 alone shows a reduction in HbA1c of only 0.5% to 1.8%, and thus is considered appropriate for patients with HbA1c of 9% or less (Ther Adv Endocrinol Metab. 2015 Feb; 6(1): 3-18). In other words, there is a limit to the blood glucose control ability that can be expected from administration of GLP-1 alone.

Accordingly, studies have been conducted on dual agonists capable of activating both GLP-1 and GIP receptors. A representative example of such a dual agonist is tirzepatide, sold under the brand name Mounjaro, which is an antidiabetic drug used to treat type 2 diabetes. Tirzepatide is administered once a week via subcutaneous injection.

Tirzepatide has the inconvenience of needing to be administered once a week via subcutaneous injection as described. To resolve this inconvenience, there is a need to develop an injectable formulation that can exert pharmacological effects of tirzepatide for one month or more by a single injection.

### [Prior Art Documents]

### [Patent Documents]

(Patent Document 1) KR 10-2021-0110349 A1

### DISCLOSURE

### Technical Problem

An object of the present invention is to provide microparticles containing tirzepatide or a pharmaceutically acceptable salt thereof and a method for producing the same.

Another object of the present invention is to provide microparticles containing tirzepatide or a pharmaceutically acceptable salt thereof, which have an increased loading amount of the drug by having an increased content of the tirzepatide or pharmaceutically acceptable salt thereof, and can exhibit the effect of releasing the tirzepatide or pharmaceutically acceptable salt thereof in a sustained manner without initial burst release and lag-time, thereby preventing excessive reduction in the bioavailability of the drug, and a method for producing the same.

Another object of the present invention is to provide microparticles containing tirzepatide or a pharmaceutically acceptable salt thereof, which may be used as an injectable formulation for subcutaneous injection, and may be contained in a reduced amount in the injectable formulation for a single injection, thereby reducing pain at the injection site and avoiding an inflammatory response, and a method for producing the same.

### Technical Solution

To achieve the above-described objects, the present invention may relate to microparticles containing tirzepatide or a pharmaceutically acceptable salt thereof, which comprise the tirzepatide or pharmaceutically acceptable salt thereof and a biodegradable polymer, wherein the biodegradable polymer comprises polylactide-co-glycolide (PLGA).

In addition, pores on the surface and inside of the microparticles may have a controlled size, so that when the microparticles are injected *in vivo*, the initial burst release of the tirzepatide or pharmaceutically acceptable salt thereof may be suppressed.

In addition, a cross-sectional SEM image (at a magnification of x2.0k) of the microparticle may show that the pores inside of the microparticle are distributed with uniform size and the inside of the microparticle has a dense structure.

In addition, an SEM image (at a magnification of x2.0k) may show that the surface of the microparticle is free of pores and the microparticle has a smooth surface.

In addition, the polylactide-co-glycolide (PLGA) may have an intrinsic viscosity of 0.2 dl/g or less and a molar ratio of lactide to glycolide of 50:50.

In addition, the tirzepatide or pharmaceutically acceptable salt thereof may be contained in an amount of 10 wt% or more based on the total weight of the microparticle.

In addition, the microparticles may have an average diameter of 30 to 70 µm.

Another aspect of the present invention for achieving the above-described objects may relate to a sustained-release injectable composition containing tirzepatide or a pharmaceutically acceptable salt thereof, which comprises the microparticles.

In addition, the injectable composition is capable of releasing the tirzepatide or pharmaceutically acceptable salt thereof *in vivo* in a sustained manner for one month or more by a single injection.

Another aspect of the present invention for achieving the above-described objects may relate to a method for producing microparticles containing the tirzepatide or pharmaceutically acceptable salt thereof, comprising: a step of preparing an oil phase solution by dissolving the tirzepatide or pharmaceutically acceptable salt thereof and a biodegradable polymer in an organic solvent; a step of preparing a water phase solution by mixing a surfactant and water; a step of forming an emulsion containing the tirzepatide or pharmaceutically acceptable salt thereof using the oil phase solution and the water phase solution; and a step of collecting and curing the formed emulsion containing the tirzepatide or pharmaceutically acceptable salt thereof, thereby producing microparticles containing the tirzepatide or pharmaceutically acceptable salt thereof, wherein the water phase solution further contains a structure modifier.

In addition, the water phase solution may contain the structure modifier at a concentration of 0.1 to 10 (w/v)%.

In addition, the step of forming an emulsion containing the tirzepatide or pharmaceutically acceptable salt thereof using the oil phase solution and the water phase solution may be performed using a microfluidic method by injecting the oil phase solution and the water phase solution into channels, respectively, and forming an emulsion at an intersection between the flow of the oil phase solution and the flow of the water phase solution.

In addition, the organic solvent may be selected from the group consisting of methanol, chloroform, chloromethane, dichloromethane, trichloroethane, ethanol, dimethyl sulfoxide, and mixtures thereof.

In addition, the oil phase solution may contain the tirzepatide or pharmaceutically acceptable salt thereof and the biodegradable polymer at a weight ratio of 1:3 to 1:15.

### Advantageous Effects

According to the present invention, it is possible to increase the loading amount of tirzepatide or a pharmaceutically acceptable salt thereof in microparticles by increasing the content of the drug in the microparticles, and exhibit the effect of releasing the tirzepatide or pharmaceutically acceptable salt thereof from the microparticles in a sustained manner without initial burst release and lag-time, thereby preventing excessive reduction in the bioavailability of the drug.

In addition, it is possible to reduce the amount of the microparticles, which are contained for a single subcutaneous injection, thereby reducing pain at the injection site and avoiding an inflammatory response.

### Brief Description of Drawings

FIG. 1 shows the results of a pharmacokinetic experiment performed using microparticles containing tirzepatide or a pharmaceutically acceptable salt thereof according to one embodiment of the present invention.
FIG. 2 shows the results of a pharmacokinetic experiment performed using microparticles containing tirzepatide or a pharmaceutically acceptable salt thereof according to one embodiment of the present invention.
FIG. 3 shows the results of a pharmacokinetic experiment performed using microparticles containing tirzepatide or a pharmaceutically acceptable salt thereof according to one embodiment of the present invention.
FIG. 4 shows the results of a pharmacokinetic experiment performed using microparticles containing tirzepatide or a pharmaceutically acceptable salt thereof according to one embodiment of the present invention.
FIG. 5 shows the results of a pharmacokinetic experiment performed using microparticles containing tirzepatide or a pharmaceutically acceptable salt thereof according to one embodiment of the present invention.
FIG. 6 shows the results of a pharmacokinetic experiment performed using microparticles containing tirzepatide or a pharmaceutically acceptable salt thereof according to one embodiment of the present invention.
FIG. 7 shows the results of a pharmacokinetic experiment performed using microparticles containing tirzepatide or a pharmaceutically acceptable salt thereof according to one embodiment of the present invention.
FIG. 8 shows SEM images of microparticles containing tirzepatide or a pharmaceutically acceptable salt thereof according to one embodiment of the present invention.
FIG. 9 shows SEM images of microparticles containing tirzepatide or a pharmaceutically acceptable salt thereof according to one embodiment of the present invention.
FIG. 10 shows SEM images of microparticles containing tirzepatide or a pharmaceutically acceptable salt thereof according to one embodiment of the present invention.
FIG. 11 shows SEM images of microparticles containing tirzepatide or a pharmaceutically acceptable salt thereof according to one embodiment of the present invention.
FIG. 12 shows SEM images of microparticles containing tirzepatide or a pharmaceutically acceptable salt thereof according to one embodiment of the present invention.
FIG. 13 shows SEM images of microparticles containing tirzepatide or a pharmaceutically acceptable salt thereof according to one embodiment of the present invention.
FIG. 14 shows SEM images of microparticles containing tirzepatide or a pharmaceutically acceptable salt thereof according to one embodiment of the present invention.
FIG. 15 shows SEM images of microparticles containing tirzepatide or a pharmaceutically acceptable salt thereof according to one embodiment of the present invention.
FIG. 16 shows SEM images of microparticles containing tirzepatide or a pharmaceutically acceptable salt thereof according to one embodiment of the present invention.
FIG. 17 shows SEM images of microparticles containing tirzepatide or a pharmaceutically acceptable salt thereof according to one embodiment of the present invention.
FIG. 18 shows SEM images of microparticles containing tirzepatide or a pharmaceutically acceptable salt thereof according to one embodiment of the present invention.
FIG. 19 shows SEM images of microparticles containing tirzepatide or a pharmaceutically acceptable salt thereof according to one embodiment of the present invention.
FIG. 20 shows the results of a pharmacokinetic experiment performed using microparticles containing tirzepatide or a pharmaceutically acceptable salt thereof according to one embodiment of the present invention.
FIG. 21 shows the results of a pharmacokinetic experiment performed using microparticles containing tirzepatide or a pharmaceutically acceptable salt thereof according to one embodiment of the present invention.
FIG. 22 shows the results of a pharmacokinetic experiment performed using microparticles containing tirzepatide or a pharmaceutically acceptable salt thereof according to one embodiment of the present invention.
FIG. 23 shows the results of a pharmacokinetic experiment performed using microparticles containing tirzepatide or a pharmaceutically acceptable salt thereof according to one embodiment of the present invention.

### Best Mode

The present invention relates to microparticles containing tirzepatide or a pharmaceutically acceptable salt thereof, which comprise the tirzepatide or pharmaceutically acceptable salt thereof and a biodegradable polymer, wherein the biodegradable polymer comprises polylactide-co-glycolide (PLGA).

### Mode for Invention

Hereinafter, embodiments of the present invention will be described in detail so that those skilled in the art can easily implement the present invention. However, the present invention may be embodied in various different forms and is not limited to the embodiments described herein.

Obesity is one of the most common nutritional disorders worldwide. According to WHO statistics, approximately 250 million people are currently classified as obese, and it is predicted that approximately 300 million people will suffer from obesity in 20 years.

The obesity refers to a state in which body fat is excessively accumulated due to an imbalance in calorie metabolism caused by excessive energy intake or decreased energy consumption. Recently, the incidence of obesity has been rapidly increasing due to the Western-style eating habits and lack of exercise of modern people.

In addition, in terms of numerical concepts, obesity is defined as a body mass index (BMI) of 25 or higher, and the BMI measurement method is an obesity measurement method that estimates the amount of body fat through the value obtained by dividing the weight (kg) by the square (m²) of the height.

Trizepatide is sold under the brand name Maunzaro, and clinical trials have shown that tirzepatide reduces body weight by an average of 18% when taken at the maximum dose. Trizepatide is a gastric inhibitory peptide (GIP)/glucagon-like peptide-1 (GLP-1) dual agonist, wherein GLP-1 can reduce appetite and increase satiety by stimulating insulin secretion to, GIP can break down adipocytes and reduce nausea.

In addition, recent clinical trials have proven that trizepatide is also effective in treating heart failure, and reduces the risk of cardiovascular events such as cardiovascular death by 38%, suggesting that trizepatide is effective not only for weight loss but also for alleviating symptoms such as heart disease that lead to serious health risks.

However, the side effects of tirzepatide are similar to those of semaglutide. Gastrointestinal symptoms such as nausea and diarrhea may be experienced. The method of administration is also similar to the method of administration of semaglutide, where the patient self-administers the drug by subcutaneous injection once a week.

To resolve the above-described problems, efforts have been continuously made to sustain the efficacy of tirzepatide or a pharmaceutically acceptable salt thereof by releasing tirzepatide or a pharmaceutically acceptable salt thereof for one month or more by a single subcutaneous injection. Accordingly, efforts have been made to develop a long-acting sustained-release formulation using a biodegradable polymer.

The formulation for sustained release of tirzepatide or a pharmaceutically acceptable salt thereof including the biodegradable polymer may be a microparticle containing the tirzepatide or pharmaceutically acceptable salt thereof.

When the microparticles containing the biodegradable polymer and tirzepatide or the pharmaceutically acceptable salt is injected *in vivo*, the microparticles are capable of releasing tirzepatide or a pharmaceutically acceptable salt thereof while degrading slowly *in vivo*.

Typically, the microparticles may degrade *in vivo* for one month or more, and thus the tirzepatide or pharmaceutically acceptable salt thereof may be released, thereby exhibiting a weight loss effect or the effect of alleviating symptoms such as heart disease.

However, these microparticles are difficult to produce with a uniform particle size, and thus it may be difficult for the microparticles to release the tirzepatide or pharmaceutically acceptable salt thereof in a sustained manner for one month.

In addition, usually, a problem may arise in that the initial burst release of tirzepatide or the pharmaceutically acceptable salt after injection occurs.

In addition, there is a problem in that the encapsulation efficiency of the tirzepatide or pharmaceutically acceptable salt thereof in the microparticles is low, or the weight ratio thereof to the biodegradable polymer is low, and thus a large amount of the microparticles need to be injected upon a single injection, causing pain during injection or inflammation at the injection site.

To prevent such problems, the present invention may relate to microparticles containing tirzepatide or a pharmaceutically acceptable salt thereof, which comprise the tirzepatide or pharmaceutically acceptable salt thereof and a biodegradable polymer, wherein the biodegradable polymer comprises polylactide-co-glycolide (PLGA).

The microparticles of the present invention are intended to be provided as a sustained-release formulation comprising tirzepatide or a pharmaceutically acceptable salt thereof as described above.

In addition, the microparticles may comprise a biodegradable polymer, wherein the biodegradable polymer may comprise polylactide-co-glycolide (PLGA).

More specifically, the polylactide-co-glycolide (PLGA) may have intrinsic viscosity of 0.2 dl/g or less and a molar ratio of lactide to glycolide of 50:50.

The microparticles comprising polylactide-co-glycolide (PLGA) as described above are capable of releasing the drug in a gradually increasing manner from the beginning without a lag time, and may exhibit the effect of releasing the tirzepatide or pharmaceutically acceptable salt thereof in a sustained manner for one month or more.

Accordingly, the microparticles of the present invention may comprise, as the biodegradable polymer, polylactide-co-glycolide (PLGA) having an intrinsic viscosity of 0.2 dl/g or less and a molar ratio of lactide to glycolide of 50:50.

In addition, the microparticles of the present invention may comprise one or two or more biodegradable polymers.

"The microparticles comprise one biodegradable polymer" may mean that the microparticles comprise a polylactide-co-glycolide (PLGA) alone, which has an intrinsic viscosity of 0.2 dl/g or less and a molar ratio of lactide to glycolide of 50:50. "The microparticles comprise two or more biodegradable polymers" may mean that the microparticles necessarily comprise a polylactide-co-glycolide (PLGA) having an intrinsic viscosity of 0.2 dl/g or less and a molar ratio of lactide to glycolide of 50:50.

Accordingly, when the microparticles comprise two or more biodegradable polymers, they may further comprise, in addition to a polylactide-co-glycolide (PLGA) having an intrinsic viscosity of 0.2 dl/g or less and a molar ratio of lactide to glycolide of 50:50, a polylactide-co-glycolide (PLGA) having a molar ratio of lactide to glycolide of 75:25, a polylactide-co-glycolide (PLGA) having a molar ratio of lactide to glycolide of 65:35, etc., and may further comprise, in addition to the polylactide-co-glycolide (PLGA), a biodegradable polymer selected from the group consisting of polylactic acid, polylactide, polylactic-co-glycolic acid, polyphosphazine, polyiminocarbonate, polyphosphoester, polyanhydride, polyorthoester, polycaprolactone, polyhydroxyvalate, polyhydroxybutyrate, and mixtures thereof.

However, if microparticles comprising a polylactide-co-glycolide (PLGA) having an intrinsic viscosity of 0.2 dl/g or less and a molar ratio of lactide to glycolide of 50:50 are produced, the microparticles may release the drug from the beginning without a lag time as described above, but initial burst release may become a problem.

In addition, the microparticle of the present invention may contain the tirzepatide or pharmaceutically acceptable salt thereof in an amount of 10 wt% or more, 11 wt% or more, 12 wt% or more, 13 wt% or more, 14 wt% or more, 15 wt% or more, and 16 wt% or more based on the total weight of the microparticle. When the content of the tirzepatide or pharmaceutically acceptable salt thereof in the microparticle is within the above-described range, the content of the drug may be increased, thereby reducing the amount of the microparticles administered by a single injection. Increasing the content of tirzepatide or the pharmaceutically acceptable salt in the microparticle may exhibit the above-described effects, but may cause initial burst release, like the case of the above-described biodegradable polymer.

That is, in the case of conventional sustained-release formulations containing tirzepatide or a pharmaceutically acceptable salt thereof as described above, if a biodegradable polymer is selected to prevent initial burst release, there is a problem in that the initial effect of the injection is not achieved due to a lag time. Even when other biodegradable polymers are used to prevent this problem, the problem of initial burst release occurs even when the content of tirzepatide or a pharmaceutically acceptable salt thereof in the particles is increased in order to reduce the amount of microparticles administered once.

In a sustained-release formulation prepared using a biodegradable polymer, the type of biodegradable polymer and the content of tirzepatide or a pharmaceutically acceptable salt thereof in the microparticle will be factors highly associated with initial burst release.

In order to resolve the above-mentioned problems and to exhibit drug release from the beginning of injection without a lag time, the present invention is intended to resolve the above-described problems by controlling the structure of the microparticle.

That is, when the microparticles of the present invention are used as an injectable composition, they exhibit drug release from the beginning without a lag time, but may exhibit the effect of releasing the tirzepatide or pharmaceutically acceptable salt thereof for one month or more without the problem of initial burst release.

Specifically, pores on the surface and inside of the microparticles of the present invention may have a controlled pore size, so that the initial burst release of the tirzepatide or pharmaceutically acceptable salt thereof when the microparticles are injected *in vivo* may be suppressed.

A cross-sectional SEM image (at a magnification of x2.0k) of the microparticle whose pore size on the surface and inside thereof has been controlled may show that the pores inside of the microparticle are distributed with uniform size and the microparticle has a dense structure. In addition, a SEM image (at a magnification of x2.0k) may show that the surface has a plurality of pores formed therein and thus the microparticle has a smooth surface.

After spherical microparticles are produced using a biodegradable polymer, it can be confirmed through a SEM image that the biodegradable polymer has a plurality of pores formed on the surface and inside thereof. The presence or absence of pores on the surface of the microparticle can be easily confirmed through a SEM image, and the pores inside of the microparticle can be confirmed through a cross-sectional SEM image of the microparticle.

In particular, when the microparticles are examined at an adjusted magnification of x2.0k, it can be confirmed that the microparticle has a plurality of pores formed on the surface and inside thereof, and that the sizes of the pores also vary. Due to the pores on the surface and inside of the microparticle as described above, the microparticles injected *in vivo* may be gradually degraded as moisture penetrates into the pores.

That is, when the microparticles are produced using a biodegradable polymer, a number of pores with various diameters may be formed on the inside and surface of the microparticle. However, the occurrence of the initial burst release and lag time described above may be determined by these various pores.

In order to resolve the above-described problem, in the present invention, the pores on the surface and inside of the microparticle are controlled to be small using a structure modifier, so that the surface has a smooth shape and the inside of the microparticle has a dense structure.

The structure modifier may be selected from the group consisting of sodium chloride (NaCl), potassium chloride (KCl), calcium chloride (CaCl₂), magnesium sulfate (MgSO₄), sodium sulfate (Na₂SO₄), mannitol, ammonium sulfate, potassium sulfate, disodium phosphate, dipotassium phosphate, trisodium phosphate, disodium citrate, trisodium citrate, and sodium succinate, and preferably may be sodium chloride (NaCl), without being limited to the above examples, and any structure modifier capable of controlling the structure of the microparticle to have the above-described structure of the present invention described above may be used without limitation.

The microparticles may have an average diameter of 30 to 70 µm, 30 to 60 µm, or 30 to 50 µm. In addition, the standard deviation of the average diameter may be 1 to 30 µm, 1 to 20 µm, 1 to 10 µm, or 1 to 7 µm. It can be confirmed that uniform particles having an average diameter within the above diameter range may be produced. When a sustained-release injectable formulation is prepared using the uniform particles, it may increase the convenience of drug administration by reducing the foreign body sensation, prevent initial burst release after *in vivo* injection, and exhibit the effect of releasing the tirzepatide or pharmaceutically acceptable salt thereof in a sustained manner.

The microparticles of the present invention may have a span value of 1.2 or less, 0.7 or less, 0.5 or less, and 0.4 or less. More specifically, the microparticles of the present invention may have a span value of 0.2 to 0.4. Microparticles having a span value within the above range mean that the diameter of the particles is very uniform. When uniform particles as described above are injected *in vivo*, they may prevent initial burst release of the tirzepatide or pharmaceutically acceptable salt thereof and exhibit the effect of releasing tirzepatide or the pharmaceutically acceptable salt in a sustained manner for one month or more.

A sustained-release injectable composition containing tirzepatide or a pharmaceutically acceptable salt thereof according to another embodiment of the present invention may comprise the microparticles.

The injectable composition is capable of releasing the tirzepatide or pharmaceutically acceptable salt thereof *in vivo* in a sustained manner for one month or more by a single injection. In addition, as described above, the injectable composition shows no initial burst release and may exhibit sustained release of the tirzepatide or pharmaceutically acceptable salt thereof from the beginning after injection without a lag time. Thus, when the injectable composition is administered by injection, it may exhibit the effect of the tirzepatide or pharmaceutically acceptable salt thereof from the beginning after injection and exhibit a sustained effect for one month or more.

The injectable composition may be prepared by mixing the microparticles of the present invention with a suspending solvent. The suspending solvent contains an isotonic agent, a suspending agent, and a solvent.

More specifically, the isotonic agent may be selected from the group consisting of D-mannitol, maltitol, sorbitol, lactitol, xylitol, sodium chloride, and mixtures thereof, and is preferably D-mannitol, but the isotonic agent is not limited to the above examples.

The suspending agent is selected from the group consisting of sodium carboxymethylcellulose, polysorbate 80, starch, starch derivatives, polyhydric alcohols, chitosan, chitosan derivatives, cellulose, cellulose derivatives, collagen, gelatin, hyaluronic acid (HA), alginic acid, algin, pectin, carrageenan, chondroitin, chondroitin sulfate, dextran, dextran sulfate, polylysine, titin, fibrin, agarose, fluran, xanthan gum, and mixtures thereof, and is preferably sodium carboxymethylcellulose and polysorbate 80, but the suspending agent is not limited to the above examples.

As the solvent, water for injection may be used, and any solvent that may be used as water for injection may be used without limitation.

A method for producing microparticles containing the tirzepatide or pharmaceutically acceptable salt thereof according to another embodiment of the present invention comprises: a step of preparing an oil phase solution by dissolving the tirzepatide or pharmaceutically acceptable salt thereof and a biodegradable polymer in an organic solvent; a step of preparing a water phase solution by mixing a surfactant and water; a step of forming an emulsion containing the tirzepatide or pharmaceutically acceptable salt thereof using the oil phase solution and the water phase solution; and a step of collecting and curing the formed emulsion containing the tirzepatide or pharmaceutically acceptable salt thereof, thereby producing microparticles containing the tirzepatide or pharmaceutically acceptable salt thereof, wherein the water phase solution may further contain a structure modifier.

The oil phase solution may be prepared by dissolving tirzepatide or a pharmaceutically acceptable salt thereof and a biodegradable polymer in an organic solvent.

The biodegradable polymer is as described above.

The organic solvent may be selected from the group consisting of methanol, chloroform, chloromethane, dichloromethane, trichloroethane, ethanol, dimethyl sulfoxide, and mixtures thereof. Preferably, the oil phase solution may be prepared by dissolving tirzepatide or a pharmaceutically acceptable salt thereof and a biodegradable polymer using, as the organic solvent, a mixed solvent consisting of dichloromethane as a solvent and methanol and dimethyl sulfoxide as co-solvents.

The oil phase solution may contain the tirzepatide or pharmaceutically acceptable salt thereof and the biodegradable polymer at a weight ratio of 1:3 to 1:15, 1:4 to 1:14, 1:5 to 1:13, 1:5 to 1:12, 1:5 to 1:11, 1:5 to 1:10, 1:5 to 1:9, 1:5 to 1:8, or 1:5 to 1:7. When the oil phase solution contains the tirzepatide or pharmaceutically acceptable salt thereof and the biodegradable polymer at a weight ratio within the above range, it may exhibit the effect of the tirzepatide or pharmaceutically acceptable salt thereof in a sustained manner for one month or more without initial burst release. In addition, as the oil phase solution contains the tirzepatide or pharmaceutically acceptable salt thereof and the biodegradable polymer at a weight ratio within the above range, it may reduce pain at injection and inhibit the occurrence of inflammation at the injection site.

The surfactant contained in the water phase solution may be any one or more selected from the group consisting of methylcellulose, polyvinylpyrrolidone, lecithin, gelatin, polyvinyl alcohol, sorbitan monooleate (e.g., Span^{™} 80, etc.), polyoxyethylene sorbitan fatty acid ester (e.g., Tween^{™} 80, etc.), polyoxyethylene castor oil derivatives, sodium lauryl sulfate, sodium stearate, ester amines, linear diamines, fatty amines, and mixtures thereof, and preferably may be polyvinyl alcohol, but the surfactant is not limited to the above examples, and any surfactant that may be prepared into a perfectly spherical emulsion may be used.

In addition, the water phase solution may further contain a structure modifier in addition to the surfactant. The structure modifier may be contained in an amount of 0.1 wt% to 10 wt%, 0.2 wt% to 9 wt%, 0.3 wt% to 8 wt%, 0.4 wt% to 7 wt%, 0.5 wt% to 6 wt%, and 0.5 wt% to 5 wt% based on the total weight of the water phase solution. If the content of the structure modifier is lower than the lower limit of the above range, the structure modification effect may be insufficient, and thus initial burst release may become a problem, and if the content of the structure modifier is more than 5 wt%, there is no difference in the effect of suppressing initial burst release by structure modification.

The final concentration of the structure modifier in the water phase solution may be 0.1 to 10 (w/v)%, 0.1 to 9 (w/v)%, 0.1 to 8 (w/v)%, 0.1 to 7 (w/v)%, 0.1 to 6 (w/v)%, 0.1 to 5 (w/v)%, or 0.5 to 5 (w/v)%. If the concentration of the structure modifier in the water phase solution is lower than the lower limit of the above range, the structure modification effect may be insufficient, and if the concentration of the structure modifier is higher than the upper limit of the above range, there is no difference in the effect of suppressing initial burst release by structure modification.

The method of preparing an emulsion using the oil phase solution and the water aqueous phase solution, separately prepared as described above, is not limited.

In the present invention, a method of producing microparticles using a microfluidic method will be described.

A microchip for using the microfluidic method may be formed on a wafer or a glass substrate. Microchannels are formed in the microchip. More specifically, the microchannels include a channel through which the oil phase solution flows, a channel through which the water phase solution flows, and a transport channel. The channel through which the oil phase solution flows and the channel through which the water phase solution flows are formed to meet each other at one point, and one end of the transport channel may be connected to a portion where the two channels are joined.

Injection portions for injecting the oil phase solution and the water phase solution are connected to the channel through which the oil phase solution flows and the channel through which the water phase solution flows, respectively, and a recovery portion for recovering a solution containing an emulsion may be connected to one end of the transport channel.

The microchannels may be formed on a material selected from the group consisting of a glass substrate, a silicon wafer and a polymer film, but the material is not limited to the above examples, and it is possible to use any material on which the microchannels may be formed.

The polymer film may be selected from the group consisting of polyimide, polyethylene, fluorinated ethylene propylene, polypropylene, polyethylene terephthalate, polyethylene naphthalate, polysulfone, and mixtures thereof, without being limited to the above examples.

As an example, aluminum is deposited on a silicon wafer using an e-beam evaporator, and photoresist is patterned on the aluminum using a photolithography technique. Thereafter, the aluminum is etched using the photoresist as a mask, the photoresist is removed, the silicon wafer is etched by deep ion reactive etching (DRIE) using the aluminum as a mask, the aluminum is removed, and then glass is anodically bonded onto the wafer and hermetically sealed, thereby fabricating the microchannels.

As the microchannels for producing the microparticles of the present invention, a 144-channel chip may be used. In the case of the microchip, the average diameter of the microchannels into which the oil phase solution and the water phase solution are to be injected is 300 µm to 500 µm, and the oil phase solution and the water phase solution may pass through resistance channels after moving through the respective channels. The average diameter of the resistance channels is 10 µm to 50 µm. After passing through the resistance channels, the oil phase solution and the water phase solution pass through a junction channel where they intersect, and the diameter of the junction channel may be 100 µm to 400 µm. After the oil phase solution and the water solution intersect each other within the junction channel to form an emulsion, they pass through a microchannel with a diameter of 150 µm to 200 µm, and then pass through a microchannel with a diameter of 200 µm to 300 µm.

However, the average diameter of the microchannels may vary depending on the range of injection pressure and flow rate conditions. In addition, the average diameter of the microchannels is closely related not only to the average diameter of the particles, but is also closely related to the injection pressure and flow rate conditions of the oil phase solution and the water phase solution.

The oil phase solution and water phase solution prepared as described above may be allowed to flow under the flow rate conditions described below through a first microchannel and a second microchannel, which have an intersection formed therebetween.

That is, the oil phase solution flows along the first microchannel, and the water phase solution flows along the second microchannel configured to form an intersection with the first microchannel, and meets the flow of the oil phase solution.

More specifically, the oil phase solution may be injected into the first microchannel at a flow rate of 100 µL/min to 300 µL/min, or 150 µL/min to 270 µL/min.

In addition, when the water phase solution may be injected into the second microchannel at a flow rate of 10,000 µL/min to 30,000 µL/min, or 15,000 µL/min to 27,000 µL/min.

As the flow rates of the oil phase solution and the water phase solution are made different from each other as described above and the flow rate of the water phase solution is increased compared to the flow rate of the oil phase solution, the water phase solution having a relatively higher flow rate compresses the oil phase solution at the point where the flow of the oil phase solution and the flow of the water phase solution meet each other, and in this case, due to repulsive force between the oil phase solution and the water phase solution, the biodegradable polymer and the tirzepatide or pharmaceutically acceptable salt thereof in the oil phase solution form spherical microparticles containing them, and more specifically, form microparticles in which the tirzepatide or pharmaceutically acceptable salt thereof is uniformly distributed in the spherical biodegradable polymer.

The temperature at which the oil phase solution and the water phase solution flow through the microchannels is also 15 to 20°C, preferably 17°C. That is, after the oil phase solution and the water phase solution flow through the microchannels and intersect each other to produce microparticles, they are constantly maintained at a low temperature of 15 to 20°C until the collected emulsion is stirred. It is possible to produce and maintain spherical particles only when the process of producing microparticles is maintained at low temperature. That is, if a low-temperature condition is not used, a problem arises in that it is difficult to produce particles having a uniform spherical shape.

The produced emulsion may be collected in a bath containing the water phase solution and stirred at 200 rpm to 300 rpm.

The water phase solution is a water phase solution used for the preparation of the emulsion, that is, a mixed solution of the surfactant and purified water. Specifically, a portion of the prepared water phase solution is injected into the microchannel, and the other portion may be transferred into the bath and used to prevent aggregation of the collected emulsion particles.

Thereafter, the temperature may be increased to 20°C to 30°C or 25°C, and the emulsion may be cured by stirring at that temperature for 0.5 to 1.5 hours or for 1 hour, thereby producing microparticles.

To remove the residual organic solvent from the microparticles, the organic solvent remaining in the emulsion may be evaporated and removed by stirring at a predetermined stirring speed at a predetermined temperature. Here, the stirring may be performed at a speed of 200 rpm 400 rpm at 35°C to 45°C for 2 hours to 4 hours. Specifically, the stirring may be performed at a speed of 200 rpm to 300 rpm at 35°C to 40°C for 2.5 hours to 3.5 hours. More specifically, the stirring may be performed at a speed of 200 rpm to 300 rpm at 40°C for 3 hours.

As the residual organic solvent is removed under the above-described stirring conditions, very small pores may be formed on the surface of the emulsion particles, enabling the production of spherical particles with a smooth surface, and the residual organic solvent may also be minimized.

Lastly, the emulsion from which the residual organic solvent has been completely removed by stirring may be washed several times with sterile filtered purified water to remove the surfactant remaining thereon, and then freeze-dried, thereby producing microparticles.

### Production Example

### Production of Microparticles Containing Tirzepatide or Pharmaceutically Acceptable Salt Thereof

Tirzepatide (manufacturer: polypeptide) and PLGA were weighed and then homogeneously dissolved in a mixed solvent prepared by adding and mixing co-solvents MeOH and DMSO with a DCM solvent, thereby preparing an oil phase solution. The mixed solvent was prepared by mixing MeOH, DMSO, and DCM at a weight ratio of 1:2:6. NaCl as a structure modifier was added to a 0.5% polyvinyl alcohol aqueous solution to a final concentration of 0.5 (w/v)%, thereby preparing a water phase solution. The oil phase solution and the water phase solution were injected into microchannels formed on a silicon wafer and allowed to flow. Here, the flow rate ratio between the oil phase solution and the water phase solution was 1:100. The temperature condition was maintained at 17°C. Microparticles produced at the intersection between the flow of the oil phase solution and the flow of the water phase solution were collected in a bath containing the water phase solution. The emulsion containing the microparticles collected in the bath was cured by stirring at a speed of 200 rpm to 300 rpm for 1 hour at 25°C. Then, the emulsion was stirred at a speed of 200 rpm to 300 rpm for 3 hours at 40°C to remove the remaining organic solvent, washed several times with sterile filtered purified water, and freeze-dried, thereby producing microparticles.

### Experimental Example 1

### Examination of Initial Burst Pattern Based on AP Ratio

When producing the microparticles of the Production Example, an oil phase solution was prepared by adjusting the weight ratio between tirzepatide and a biodegradable polymer as shown in Table 1 below, a water phase solution containing no structure modifier was prepared, and microparticles were produced using the oil phase solution and the water phase solution. An injectable composition was prepared by suspending the microparticles in water for injection, and pharmacokinetic evaluation was performed using the injectable composition. The test conditions are as follows:
- Male SD rats, 6 weeks old
- Ten SD rats per group (a total of 40 SD rates) were used, and the average value of plasma tirzepatide concentrations was calculated at each measurement time.
- Subcutaneous administration (1.24 mg/head)

**[Table 1]**

| Tirzepatide | Biodegradable polymer | Drug loading amount |
|---|---|---|
| 1 | 10 | 9.1% |
| 1 | 7 | 12.5% |
| 1 | 5 | 16.7% |

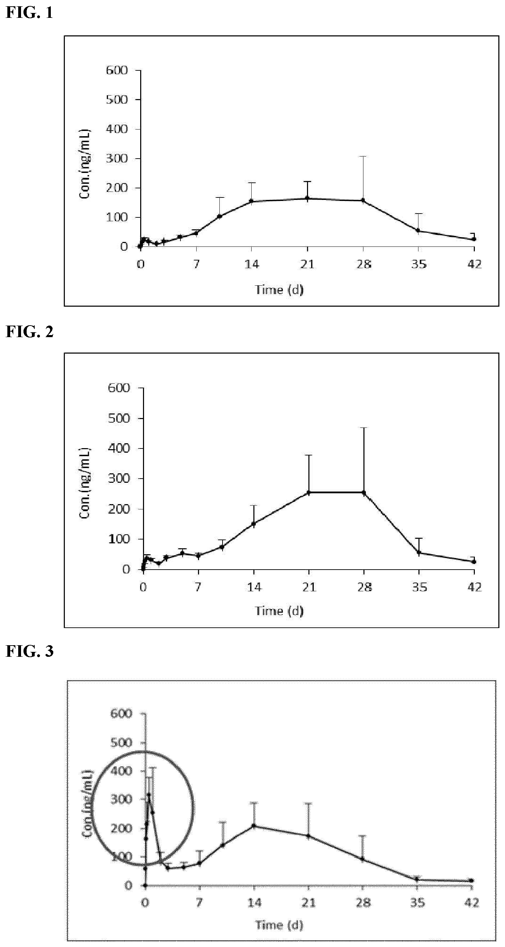

FIGS. 1 to 3 relate to the case in which PDLG7502A (with a molar ratio of lactide to glycolide of 75:25) was used as the biodegradable polymer. FIG. 1 relates to the case in which the weight ratio between tirzepatide (manufacturer: polypeptide) and the biodegradable polymer was 1:10 (drug loading amount: 9.1%), FIG. 2 relates to the case in which the weight ratio between tirzepatide (manufacturer: polypeptide) and the biodegradable polymer was 1:7 (drug loading amount: 12.5%), and FIG. 3 relates to the case in which the weight ratio between tirzepatide (manufacturer: polypeptide) and the biodegradable polymer was 1:5 (drug loading amount: 16.7%). Referring to FIGS. 1 to 3, it was confirmed that initial burst release occurred as the drug content increased.

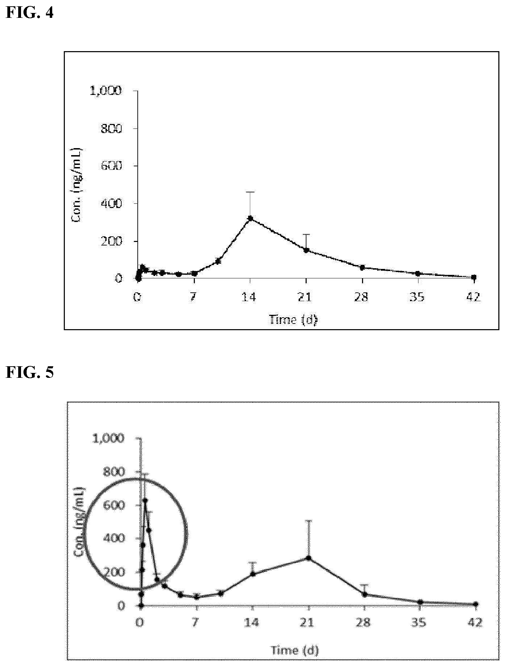

Additionally, an oil phase solution was prepared using PDLG5002A (with a molar ratio of lactide to glycolide of 50:50) and PDLG7502A (with a molar ratio of lactide to glycolide of 75:25) mixed at a weight ratio of 1:3 as biodegradable polymers such that the weight ratio between tirzepatide (manufacturer: polypeptide) and PLGA was 1:7 (FIG. 4) or 1:5 (FIG. 5), and a water phase solution containing no structure modifier was prepared. Using the oil phase solution and the water phase solution, microparticles were produced.

Referring to FIGS. 4 and 5, it was confirmed that, when PDLG5002A was further contained and the weight ratio between tirzepatide and the biodegradable polymers was 1:5, a larger initial burst release than when PDLG7502A alone was used as the biodegradable polymer (FIG. 3) occurred.

Additionally, different types of biodegradable polymers were used to examine whether initial burst release was affected by the type of biodegradable polymer. An oil phase solution was prepared using PDLG7504A (with a molar ratio of lactide to glycolide of 75:25) or PDL02A (PLA polymer) as a biodegradable polymer such that the weight ratio between tirzepatide and the biodegradable polymer was 1:5, and a water phase solution no containing structure modifier was prepared. Using the oil phase solution and the water phase solution, microparticles were produced.

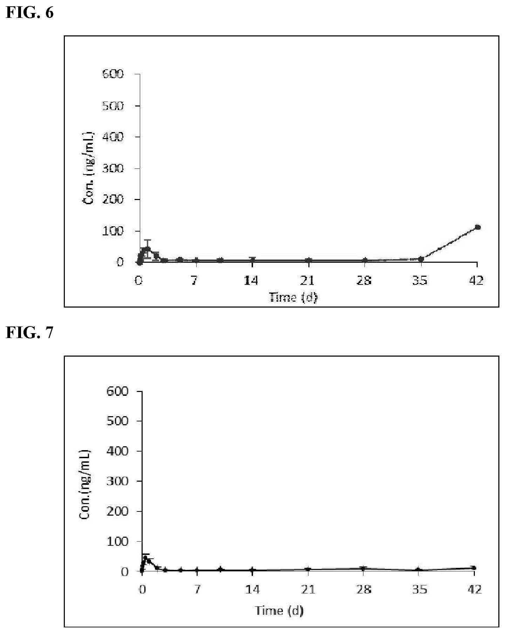

The test results are shown in FIGS. 6 and 7.

Referring to the test results, it was confirmed that, when PDLG7504A or PDL02A was used as the biodegradable polymer, no initial burst release occurred, but after initial release occurred, almost no release of tirzepatide was observed.

From the above experiments, it was confirmed that, when PLGA with a molar ratio of lactide to glycolide of 50:50 was used as the biodegradable polymer, or when the weight ratio between tirzepatide to PLGA was 1:5 (drug loading amount: 16.7%), the problem of initial burst release occurred.

### Experimental Example 2

### Examination of Structure of Microparticles Based on Presence or Absence of Structure Modifier

Microparticles were produced in the same manner as in the Production Example. An oil phase solution was prepared using PDLG5002A (with a molar ratio of lactide to glycolide of 50:50) and PDLG7502A (with a molar ratio of lactide to glycolide of 75:25) mixed at a weight ratio of 1:3 as biodegradable polymers such that the weight ratio between tirzepatide (manufacturer: polypeptide) and PLGA was 1:5, and then a water phase solution containing or not containing NaCl was prepared. Using the oil phase solution and the water phase solution, microparticles were produced.

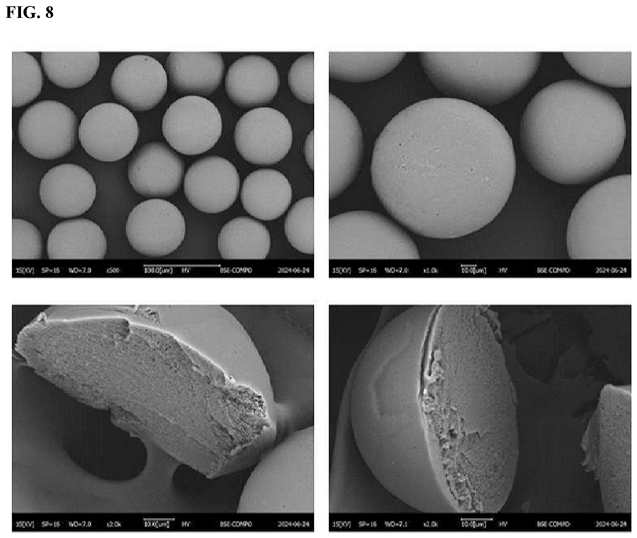

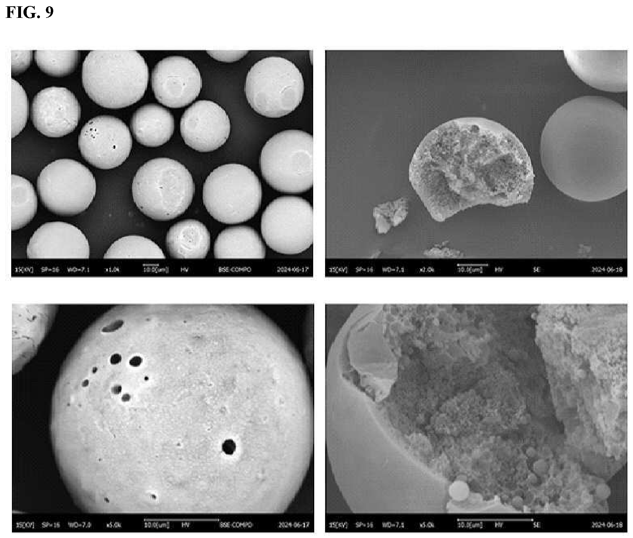

FIG. 8 shows SEM images of microparticles containing a structure modifier, like the microparticles of the Production Example of the present invention, and FIG. 9 shows SEM images of microparticles produced using the water phase solution containing no structure modifier.

Referring to FIG. 8, it can be confirmed that the surface of the particles is very uniform and has almost no pores, and that the cross-sectional structure of the particles is very dense.

On the other hand, referring to FIG. 9, it can be confirmed that the surface of the particles has large pores formed thereon and the cross-sectional structure of the particles is not dense. In addition, it can be confirmed that, during the process of cutting the microparticles to form the cross-section, the particles were not cut uniformly and broke easily.

### Experimental Example 3

### Examination of Structure of Microparticle Based on Content of Structure Modifier

Microparticles were produced in the same manner as in the Production Example. An oil phase solution was prepared using PDLG5002 (with a molar ratio of lactide to glycolide of 50:50) and PDLG7502 (with a molar ratio of lactide to glycolide of 75:25) mixed at a weight of 1:3 as biodegradable polymers such that the weight ratio between tirzepatide (manufacturer: polypeptide) and PLGA was 1:5, and then a water phase solution containing NaCl at a final concentration of 5 (w/v)%, 0.5 (w/v)% or 0.1 (w/v)% was prepared. Using the oil phase solution and the water phase solution, microparticles were produced.

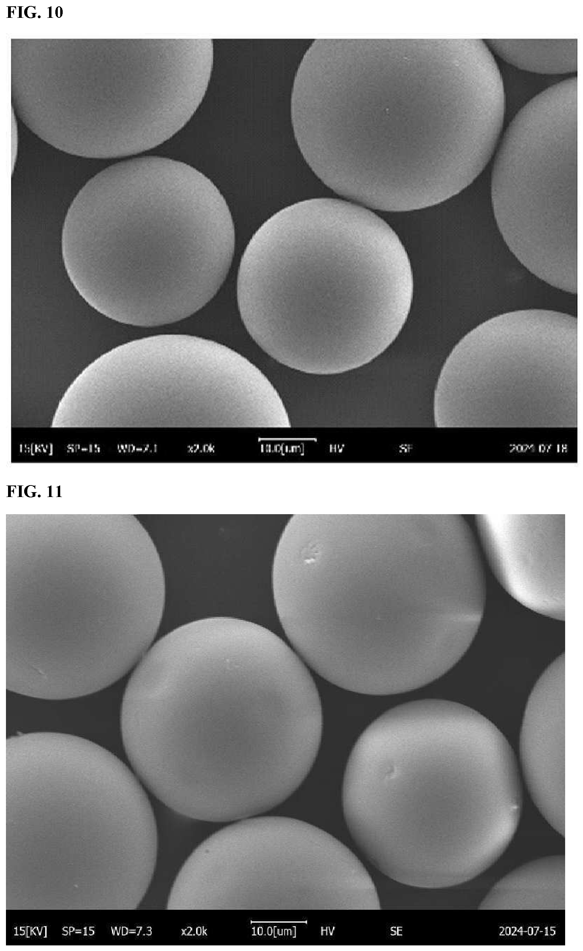

The results are shown in FIGS. 10 to 15.

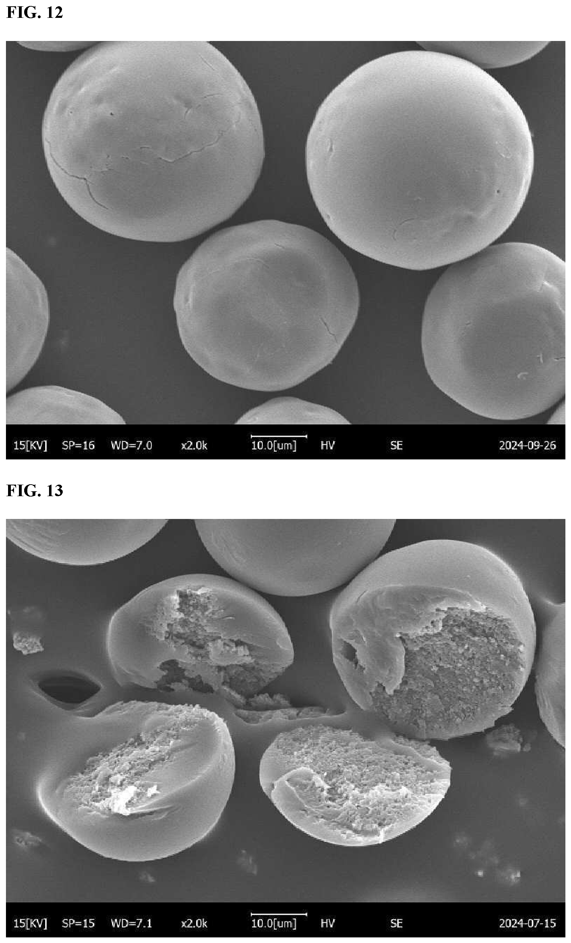

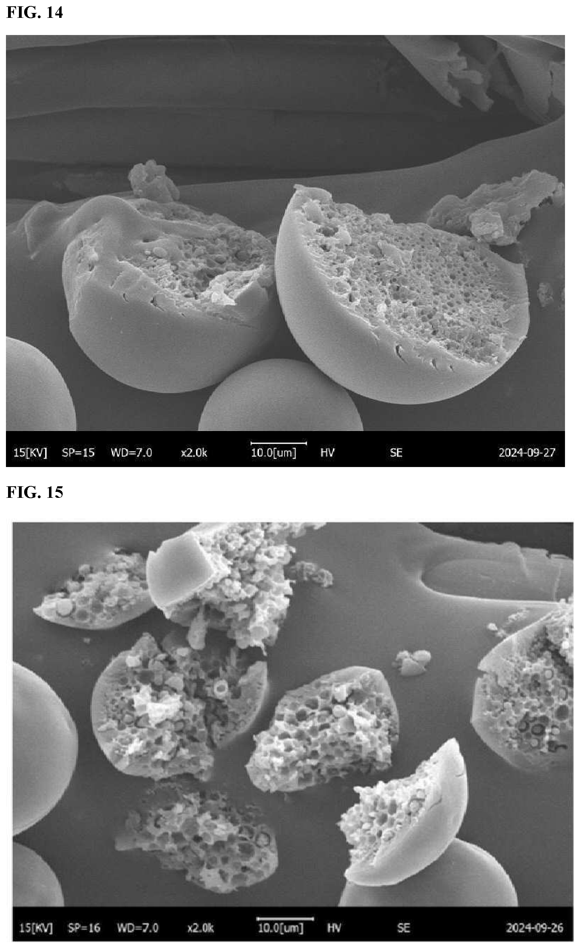

FIGS. 10 and 13 relate to the case in which the final concentration of NaCl in the water phase solution was 5 (w/v)%, FIGS. 11 and 14 relate to the case in which the final concentration of NaCl in the water phase solution was 0.5 (w/v)%, and FIGS. 12 and 15 relate to the case in which the final concentration of NaCl in the water phase solution was 0.1 (w/v)%. FIGS. 10 to 15 show SEM images at a magnification of x2.0k.

FIGS. 10 to 12 show images of the surfaces of microparticles. Referring to FIGS. 10 and 11, it can be confirmed that the surfaces of the microparticles had a smooth spherical shape, and referring to FIG. 12, it can be confirmed that the surfaces of the particles had an irregular shape. The reason the surface appears irregular and bumpy is that it appears as a hollow shape depending on the pores, or the surface was not uniformly formed.

FIGS. 13 to 15 show cross-sectional images of microparticles. It can be confirmed that, as the content of the structure modifier increased, the pores inside of the microparticles are smaller and denser. On the other hand, it can be confirmed that, when the final concentration of the structure modifier in the aqueous solution was 0.1 (w/v)%, large pores were formed large, and thus during the process of cutting the particles, the particles broke rather than being cut.

Additionally, an oil phase solution was prepared using RG502H (with a molar ratio of lactide to glycolide of 50:50), RG504H (with a molar ratio of lactide to glycolide of 50:50), and RG653H (with a molar ratio of lactide to glycolide of 65:35) mixed at a weight ratio of 1:1:1 as biodegradable polymers such that the weight ratio between tirzepatide (manufacturer: polypeptide) and PLGA was 1:5, and then water phase solutions containing NaCl at final concentrations of 5 (w/v)%, 0.5(w/v)% and 0.1(w/v)% and not containing NaCl were prepared. Using the water phase solution and each of the water phase solutions, microparticles were prepared.

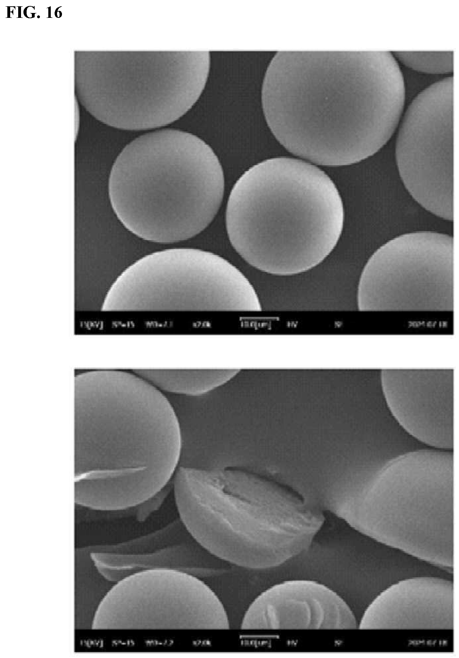

FIGS. 16 to 19 show the results of comparing the internal and surface structures of microparticles depending on the difference in concentration of NaCl in the water phase solution.

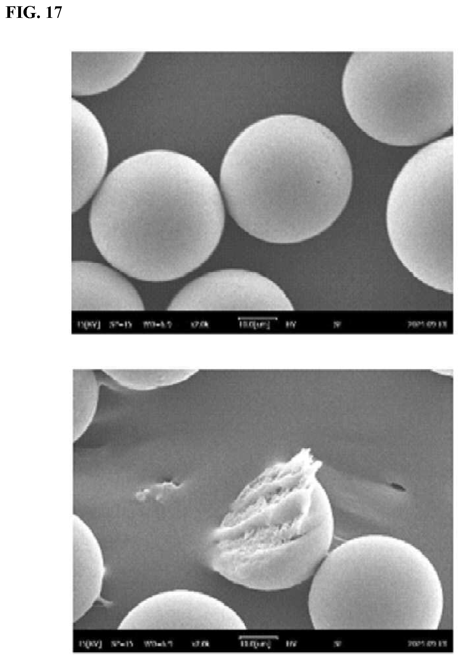

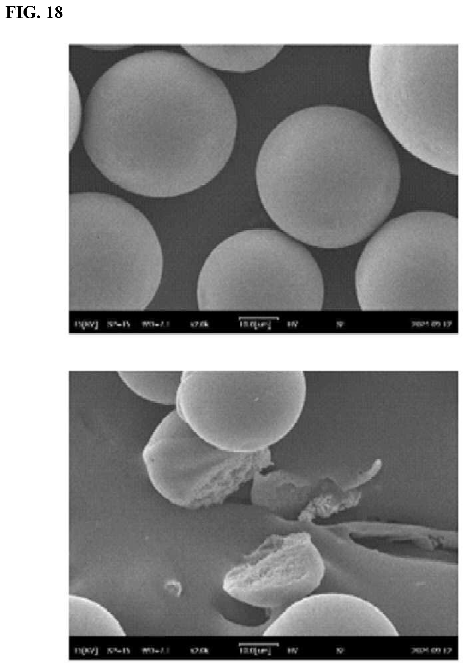

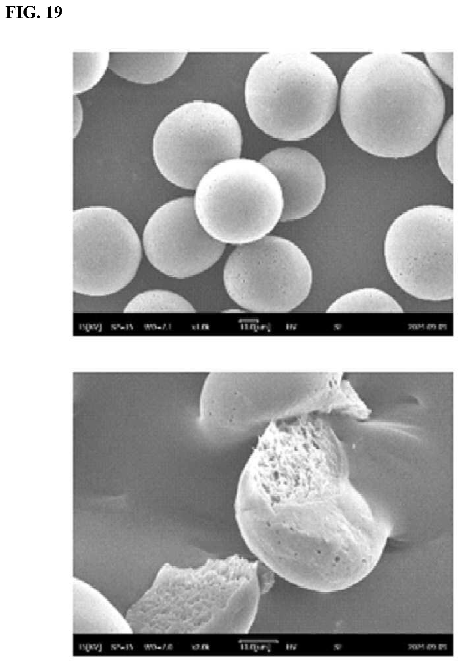

FIG. 16 relates to the case in which the final concentration of NaCl in the water phase solution was 5 (w/v)%, FIG. 17 relates to the case in which the final concentration of the final concentration of NaCl in the water phase solution was 0.5 (w/v)%, FIG. 18 relates to the case in which the final concentration of NaCl in the water aqueous solution was 0.1 (w/v)%, and FIG. 19 relates to the case in which the water phase solution not containing NaCl was used.

It can be confirmed that, even when the type of biodegradable polymer was kept the same, the surface and cross-sectional structures of the particles did differ depending on whether the water phase solution contained NaCl and on the difference in concentration when NaCl was contained.

### Experimental Example 4

### Examination of Whether Initial Burst Release is Suppressed by Containing Structure Modifier

An oil phase solution was prepared using RG502H (with a molar ratio of lactide to glycolide of 50:50), RG504H (with a molar ratio of lactide to glycolide of 50:50), and RG653H (with a molar ratio of lactide to glycolide of 65:35) mixed at a weight ratio of 1:1:1 as biodegradable polymers such that the weight ratio between tirzepatide (manufacturer: polypeptide) and PLGA was 1:5, and then a water phase solutions containing NaCl at a final concentration of 5 (w/v)% or 0.1(w/v)% was prepared. Using the water phase solution and the water phase solution, microparticles were produced. An injectable composition was prepared by suspending the microparticles in water for injection, and pharmacokinetic evaluation was performed using the injectable composition. The test conditions are as follows:
- Male SD rats, 7 weeks old
- Ten SD rats per group (a total of 40 SD rates) were used, and the average value of plasma tirzepatide concentrations was calculated at each measurement time.
- Subcutaneous administration (1.24 mg/head)

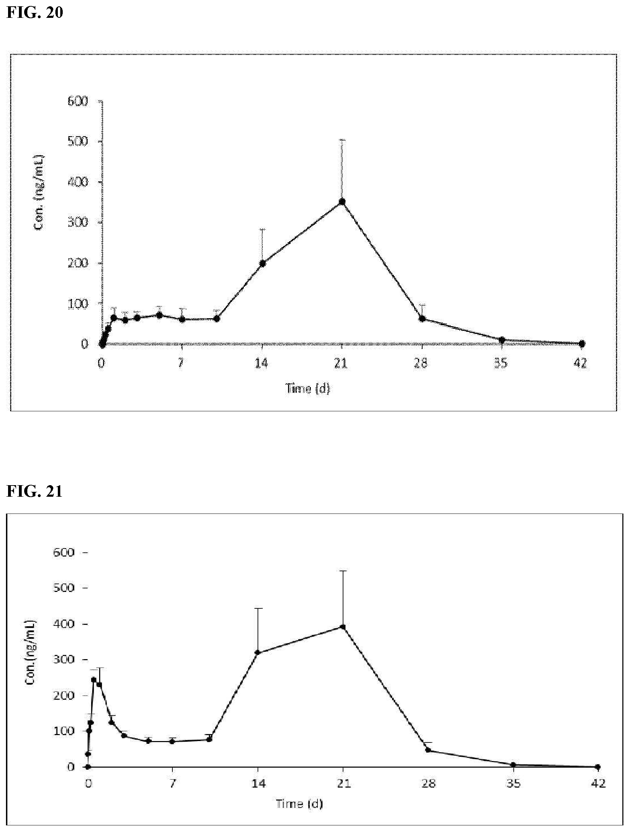

The test results are shown in Table 2 below and FIGS. 20 and 21.

**[Table 2]**

| Group | Formulation | AP ratio | NaCl concentration | Cmax (ng/ml) | AUC_{0∼28day} (hr*ng/ml) | Tmax (day) |
|---|---|---|---|---|---|---|
| 3^{rd} PK G8 | 502H/504H/6 53H=1/1/1 | 1:5 | 5% | 377.14 | 107554.89 | 21.0 |
| 4^{th} PK G2 | 502H/504H/6 53H=1/1/1 | 1:5 | 0.1% | 392.54 | 129576.74 | 21.0 |

From the above test results, it was confirmed that initial burst release occurred was dependent on the concentration of NaCl in the water phase solution. However, when the water phase solution containing NaCl at a concentration of 5 (w/v)% was used, there was a slight decrease in bioavailability compared to the water phase containing NaCl at a concentration of 0.1 (w/v)% was used, but the difference was not significant.

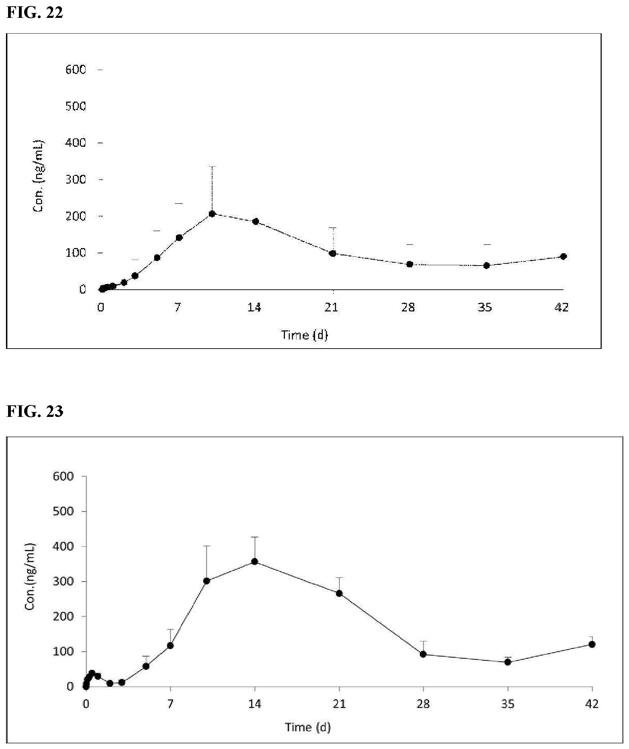

In addition, Table 3 below and FIGS. 22 and 23 show the results of the pK evaluation for the case in which the water phase solution containing 5(w/v)% or 0.5(w/v)% NaCl was used.

**[Table 3]**

| Group | Formulation | AP ratio | NaCl concentration | Cmax (ng/ml) | AUC_{0∼28day} (hr*ng/ml) | Tmax (day) |
|---|---|---|---|---|---|---|
| 3^{rd} PK G4 | 5002/7502=1/3 | 1:5 | 5% | 212.57 | 102104.25 | 10 |
| 4^{th} PK G5 | 5002/7502=1/3 | 1:5 | 0.5% | 356.35 | 244950.05 | 14 |

The experimental results in Table 3 above were obtained under the same experimental conditions as in Table 2 above. It was confirmed that there were differences in Cmax, AUC, and Tmax values due to differences in biodegradable polymers, but when the water phase solution containing 0.5% NaCl was used, the effect of suppressing initial burst release appeared, similar to when the water phase solution containing 5% NaCl was used. However, as shown in Table 3 above, it can be confirmed that the AUC decreased as the concentration of NaCl increased, suggesting that the structure can be controlled by NaCl. Initial burst can be controlled by the control of the structure as described above, but controlling the size of the pores, whether the pores are created, etc. may affect the degree of degradation of the biodegradable polymer *in vivo*. For this reason, if a water phase solution containing NaCl at a concentration of more than 5% is used, a problem may arise in that the bioavailability of tirzepatide or a pharmaceutically acceptable salt thereof becomes excessively low, and thus the effect resulting from the release of tirzepatide or a pharmaceutically acceptable salt thereof may be insufficient.

Although the preferred embodiments of the present invention have been described in detail above, the scope of the present invention is not limited thereto, and various modifications and improvements made by those skilled in the art using the basic concept of the present invention as defined in the appended claims also fall within the scope of the present invention.

### Industrial Applicability

The present invention relates to microparticles containing tirzepatide or a pharmaceutically acceptable salt thereof and a method for producing the same.

## Claims

1. A microparticle containing tirzepatide or a pharmaceutically acceptable salt thereof, which comprises the tirzepatide or pharmaceutically acceptable salt thereof and a biodegradable polymer
wherein the biodegradable polymer comprises polylactide-co-glycolide (PLGA).

2. The microparticle of claim 1, wherein pores on a surface and inside of the microparticle have a controlled size, so that when the microparticle is injected *in vivo*, initial burst release of the tirzepatide or pharmaceutically acceptable salt thereof is suppressed.

3. The microparticle of claim 2, wherein a cross-sectional SEM image (at a magnification of x2.0k) of the microparticle shows that the pores inside of the microparticle are distributed with uniform size and the inside of the microparticle has a dense structure.

4. The microparticle of claim 2, wherein a SEM image (at a magnification of x2.0k) shows that the surface of the microparticle is free of pores and thus the microparticle has a smooth surface.

5. The microparticle of claim 1, wherein the polylactide-co-glycolide (PLGA) has an intrinsic viscosity of 0.2 dl/g or less and a molar ratio of lactide to glycolide of 50:50.

6. The microparticle of claim 1, wherein the tirzepatide or pharmaceutically acceptable salt thereof is contained in an amount of 10 wt% or more based on a total weight of the microparticle.

7. The microparticle of claim 1, wherein the microparticle has an average diameter of 30 to 70 µm.

8. A sustained-release injectable composition containing tirzepatide or a pharmaceutically acceptable salt thereof, which comprises the microparticle of any one of claims 1 to 7.

9. The sustained-release injectable composition of claim 8, which releases the tirzepatide or pharmaceutically acceptable salt thereof *in vivo* in a sustained manner for one month or more by a single injection.

10. A method for producing microparticles containing the tirzepatide or pharmaceutically acceptable salt thereof, comprising:
a step of preparing an oil phase solution by dissolving the tirzepatide or pharmaceutically acceptable salt thereof and a biodegradable polymer in an organic solvent;
a step of preparing a water phase solution by mixing a surfactant and water;
a step of forming an emulsion containing the tirzepatide or pharmaceutically acceptable salt thereof using the oil phase solution and the water phase solution; and
a step of collecting and curing the formed emulsion containing the tirzepatide or pharmaceutically acceptable salt thereof, thereby producing microparticles containing the tirzepatide or pharmaceutically acceptable salt thereof,
wherein the water phase solution further contains a structure modifier.

11. The method of claim 10, wherein the water phase solution contains the structure modifier at a concentration of 0.1 to 10 (w/v)%.

12. The method of claim 10, wherein the step of forming an emulsion containing the tirzepatide or pharmaceutically acceptable salt thereof using the oil phase solution and the water phase solution is performed using a microfluidic method by injecting the oil phase solution and the water phase solution into channels, respectively, and forming an emulsion at an intersection between the flow of the oil phase solution and the flow of the water phase solution.

13. The method of claim 10, wherein the organic solvent is selected from the group consisting of methanol, chloroform, chloromethane, dichloromethane, trichloroethane, ethanol, dimethyl sulfoxide, and mixtures thereof.

14. The method of claim 10, wherein the oil phase solution contains the tirzepatide or pharmaceutically acceptable salt thereof and the biodegradable polymer at a weight ratio of 1:3 to 1:15.
